Europäisches Patentamt

European Patent Office

Office européen des brevets

(11) Veröffentlichungsnummer: **0 292 888 B1**

(12) # EUROPÄISCHE PATENTSCHRIFT

(45) Veröffentlichungstag der Patentschrift: **12.08.92**

(51) Int. Cl.$^5$: **B01D 29/50**, B01D 24/00, B01D 39/00

(21) Anmeldenummer: **88108083.2**

(22) Anmeldetag: **20.05.88**

(54) **Verfahren zur Abtrennung fester Partikel verschiedener Grösse aus viskosen Flüssigkeiten.**

(30) Priorität: **27.05.87 DE 3717902**

(43) Veröffentlichungstag der Anmeldung:
**30.11.88 Patentblatt 88/48**

(45) Bekanntmachung des Hinweises auf die
Patenterteilung:
**12.08.92 Patentblatt 92/33**

(84) Benannte Vertragsstaaten:
**AT DE FR GB NL**

(56) Entgegenhaltungen:
EP-A- 0 123 316    WO-A-80/00222
DE-A- 3 135 813    DE-B- 1 300 090
GB-A- 1 260 591    US-A- 3 722 686

(73) Patentinhaber: **WINTERSHALL AKTIENGESELL-
SCHAFT
Postfach 10 40 20 Friedrich-Ebert-Strasse
160
W-3500 Kassel(DE)**

(72) Erfinder: **Lindörfer, Walter, Dr.
Christian-Beyer-Strasse 16
W-3500 Kassel(DE)**
Erfinder: **Sewe, Kai-Udo, Dr.
Zuckmayerstrasse 6
W-2847 Barnstorf(DE)**
Erfinder: **Jahn-Held, Wilhelm, Dr.
Schöne Aussicht 8
W-3513 Staufenberg(DE)**
Erfinder: **Ziebholz, Burkhard, Dr.
Fasanenstrasse 2
W-3300 Braunschweig(DE)**
Erfinder: **Wagner, Fritz, Prof. Dr.
Hohe Wiese 2
W-3300 Braunschweig-Stöckheim(DE)**

(74) Vertreter: **Springer, Hans Jörg, Dr. et al
BASF Aktiengesellschaft Patentabteilung
Carl-Bosch-Strasse 38
W-6700 Ludwigshafen(DE)**

Rank Xerox (UK) Business Services

EP 0 292 888 B1

## Beschreibung

Die Erfindung betrifft ein Verfahren zur Abtrennung fester Partikel verschiedener Größe aus viskosen Flüssigkeiten, beispielsweise Biomasse aus Flüssigkeiten biotechnologischer Prozesse, mittels Druckfiltration.

Bei der Aufarbeitung viskoser Polysaccharidlösungen aus biotechnologischen Prozessen gibt es ein grundlegendes Problem: die Abtrennung der Biomasse von der viskosen Flüssigphase. Im Falle des durch einen Pilzstamm ATCC 15205 gebildeten Polysacchardis, eines $\beta$-D-1,3 Glucans, ergeben sich zusätzliche Probleme durch die Tatsache, daß das Produkt zu einem nicht unbeträchtlichen Teil am Mycel haftet und Zellwandbestandteilen strukturanalog ist.

Die native Kulturbrühe wurde bisher derart aufgearbeitet, daß man dieselbe stark verdünnte, dann durch herkömmliches Filtermaterial filtrierte oder zentrifugierte und das Filtrat wieder aufkonzentrierte. Auf den Verdünnungsschritt kann dabei nicht verzichtet werden, da die unverdünnte Kulturbrühe (eine inhomogene Mischung aus Mycel, Polysaccharid, Medienbestandteilen und Wasser) jedes untersuchte konventionelle Filtermedium nach kürzester Zeit verstopft, bzw. wegen hoher Viskosität eine Sedimentation der Biomasse durch Zentrifugieren unmöglich macht. Eine Auftrennung in einem 2-Phasen-System ist wegen der Strukturanalogie Produkt-Zellwand ebenfalls unmöglich.

Trotz Verdünnung sind die Membranstandzeiten bei den genannten Filtrationen nur kurz. Ein effektiveres Verfahren ohne größeren apparativen Aufwand ist aus der einschlägigen Literatur nicht bekannt.

Ausgehend vom Stand der Technik, siehe Ullmanns Encyclopädie der Technischen Chemie, 4. Auflage, Band 2, Seiten 156/157, liegt dieser Erfindung die Aufgabe zugrunde, aus möglichst unverdünnten, nativen Kulturbrühen d.h. strukturviskosen Suspensionen die Biomasse abzutrennen.

Diese Aufgabe wird erfindungsgemäß durch die Merkmale des Anspruchs 1 gelöst.

Weitere erfindungsgemäße Merkmale der Erfindung sind Gegenstand der Unteransprüche.

Der Druck wird dabei auf die Gasphase über der zu filtrierenden Flüssigkeit oder direkt auf die Flüssigphase ausgeübt und beträgt 1 bis 10 bar. Die Filterschichten für die Abtrennung grober Partikel bestehen aus einem dreidimensionalen Netzwerk aus Metallgewebe, Kunststoffgeflecht oder Cellulosemasse mit einer mittleren Porenweite von 1 bis 3 mm und von ausreichender Tiefe (10 bis 100 mm). Beispielsweise beträgt die Dichte eines solchen Kunststoffilters bis zu 80 kg/m$^3$, die der Metallausführung ist entsprechend höher.

Für die Abtrennung feinerer Teilchen wird anschließend offenporiger Polyurethanschaumstoff (durchschnittl. Porenweite 0,2 bis 0,8 mm, Dichte bis zu 80 kg/m$^3$) und zuletzt Seidengaze (Maschenweite 0,006 bis 0,2 mm) oder ähnliche Filterflächen aus natürlichen oder synthetischen Stoffen verwendet. Die Porenweite des Schaumstoffs kann durch Kompression desselben verkleinert werden.

Diese Filtertechnik ist eine Kombination aus Tiefen- und Oberflächenfiltration. In den oberen, zuerst durchströmten Filterschichten bleibt die Biomasse im Filter hängen, an der Seidengaze bzw. den Filterflächen wird die schon vorgereinigte Suspension einer Oberflächenfiltration unterzogen.

Die Filterapparatur sollte so beschaffen sein, daß die Filterfläche möglichst groß ist. Die Strömung im Inneren muß durch konstruktive Maßnahmen so beschaffen sein, daß die Suspension sich nicht an den Filterschichten vorbeidrücken kann.

Das System ist flexibel und wird durch Austausch der Filterschichten an unterschiedliche Problemstellungen angepaßt. Es hat sich besonders bei sehr inhomogenen Kulturbrühen mit hohem Mycelgehalt (bis 10 gTG/l) und hoher Polymerkonzentration (bis 12 gTG/l) bewährt, wie sie aus Schüttelkolbenkultivierungen erhalten werden (TG = Trockengewicht).

Es versteht sich, daß das Verfahren auch auf anderen Gebieten Anwendung findet, bei denen es um die Aufarbeitung viskoser Flüssigkeiten geht, wie beispielsweise

- Produktion von Celluloid
- Produktion von Celluloseether
- Produktion von Cellulosechemiefasern
- Produktion von Fetten und Ölen
- Produktion von Gelatine
- Produktion von natürlichen und synthetischen Harzen
- Produktion von Klebstoffen und Dichtungsmassen
- Produktion von Pektin
- Produktion von Stärke
- Produktion von Schmierstoffen
- Produktion von anderen Polysacchariden (Agar, Alginat, Corrageenan, Dextran)

Das erfindungsgemäße Verfahren wird im folgenden anhand einer Zeichnung und an verschiedenen Beispielen näher beschrieben.

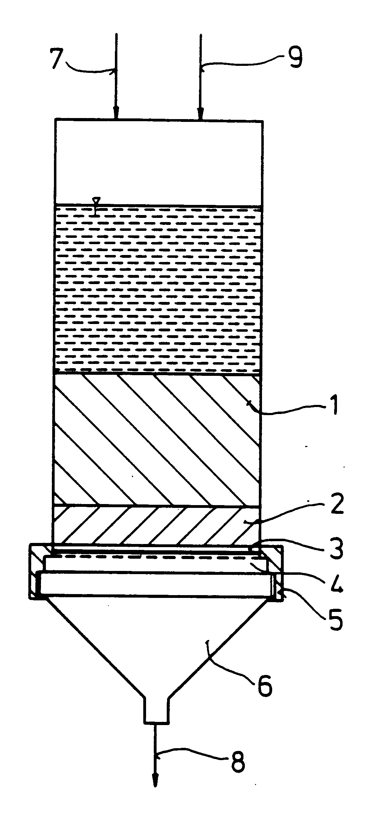

Die Zeichnung zeigt einen Schnitt durch eine labormäßige Druckfiltereinheit, mittels der verschiedene Kulturbrühen gefiltert wurden.

In der Druckfiltereinheit sind von oben nach unten in Strömungsrichtung - u.U. auch nebeneinander - drei verschiedene Filterschichten 1, 2 und 3 angeordnet. Entsprechend bekanntem Apparatebau sind diese Filterschichten auf einer Filteraufnahmeeinheit 4 angeordnet, und werden in die Druckfiltereinheit von unten eingeschoben. Mittels einer Überwurfmutter 5 wird der konische Filterauslauf 6 mit der Druckfiltereinheit verbunden und gleichzeitig die Filteraufnahmeeinheit fest in die Druckfiltereinheit eingebunden. Der Zulauf der Kulturbrühe erfolgt über die Leitung 7 und der Filtratablauf über die Leitung 8. Über die Leitung 9 wird mittels Luft oder Inertgas der notwendige Druck auf die Gasphase über der Kulturbrühe oder direkt auf die Kulturbrühe aufgebracht. Durch die beschriebene Druckfilterkonstruktion lassen sich die Filterschichten rasch und problemlos wechseln; dies ist jedoch gängiger Apparatebau und nicht erfindungsrelevant. Es werden zweckmäßigerweise zwei Druckfiltereinheiten parallel angeordnet, so daß stets eine Einheit in Betrieb ist, während gleichzeitig bei der anderen die Filterschichten gereinigt bzw. gewechselt werden. Des weiteren ist es verfahrenstechnisch vorteilhaft, die Filterstufen mittels Rückspülung zu reinigen.

Beispiel 1

Dreistufige Druckfiltration einer viskosen, inhomogenen Zellsuspension

Eine durch Züchtung von Sclerotium rolfsii ATCC 15 205 erhaltene viskose, wäßrige Zellsuspension setzt sich wie folgt zusammen:
Inhomogene Zellgrößenverteilung von 0,005 mm (Hyphenbruchstücke bis 1 cm Pellets) Durchmesser
    Zelltrockenmasse:
4,53 g/l
Homoglucangehalt der wäßrigen Lösung:
5,12 g/l
Viskosität der Zellsuspension:
ca. 1200 mPas bei einer Scherrate $D = 9s^{-1}$ bei 20°C.
    1 Liter dieser Zellsuspension wird in die Druckfiltereinheit gemäß Zeichnung eingeführt und bei 3 bar Überdruck und 20°C einer Druckfiltration unterworfen.

Filteraufbau

| | |
|---|---|
| Filterstufe 1: | Dreidimensionales Kunststoffgewebe mit einer mittleren Porenweite von 2 mm und einer Schichthöhe von 40 mm |
| Filterstufe 2: | Polyurethanschaumstoff (Dichte: 60 kg/m$^3$, Porenweite: 0,55 mm, Schichthöhe: 10 mm) |
| Filterstufe 3: | Seidengaze (Porenweite: 0,006 mm, Schichthöhe: 0,5 mm) |
| Filterfläche: | 10,7 cm$^2$ |

Ergebnis:

Im wasserklaren Filtrat kann keine Zellmasse nachgewiesen werden, das Filtrat besitzt eine Viskosität bei 20°C und $D = 9s^{-1}$ von 738 mPas und einen Homoglucangehalt von 10,0 g/l, entsprechend einem Filtrationsverlust an Homoglucan von 2,14 Gew.%. Die Filtrationsrate beträgt 530 l/m$^2$ h.

Beispiel 2
Beispiel nach dem Stand der Technik

Einstufige Druckfiltration einer viskosen, homogenen Zellsuspension

Eine durch Züchtung von Sclerotium rolfsii ATCC 15 205 erhaltene inhomogene wäßrige Zellsuspension wird mechanisch homogenisiert, so daß die Zellgrößenverteilung bei 0,002 bis 0,005 mm Durchmesser liegt.
    Zelltrockenmasse:
2,38 g/l
Homoglucangehalt der wäßrigen Lösung:
2,93 g/l
Viskosität der Zellsuspension:
420 mPas bei einer Scherrate $D = 9s^{-1}$ und 20°C.
    1 Liter dieser homogenen Zellsuspension wird in die Druckfiltereinheit gemäß Zeichnung eingeführt und bei 4 bar Überdruck und 20°C einer Druckfiltration unterworfen.

Filteraufbau

| | |
|---|---|
| Filterstufe 2: | Polyurethanschaumstoff (Dichte: 60 kg/m$^3$, Porenweite: 0,25 mm, Schichthöhe: 50 mm) |
| Filterfläche: | 10,7 cm$^2$ |

Ergebnis:

Leicht opaleszierendes Filtrat mit einem Zellpartikeltrockengehalt von 0,013 Gew.%. Das Filtrat besitzt eine Viskosität bei 20°C und $D = 9s^{-1}$ von 110 mPas, der Homoglucangehalt beträgt 2,15 g/l, entsprechend einem Filtrationsverlust an Homoglucan von 26,6 Gew.%. Die Filtrationsrate beträgt 1703 l/m$^2$h.

Beispiel 3

Zweistufige Druckfiltration einer viskosen, homogenen Zellsuspension

Eine durch Züchtung von Sclerotium Glucanicum CBS 52071 in einem begasten, mechanisch gerührten 50 l Bioreaktor erhaltene viskose, wäßrige Zellsuspension setzt sich wie folgt zusammen:
Homogene Zellgrößenverteilung von 0,002 mm (Mycelbruchstücke) Durchmesser
Zelltrockenmasse:
0,81 g/l
Homoglucangehalt:
4,25 g/l
Viskosität der Zellsuspension:
380 mPas bei einer Scherrate $D = 9s^{-1}$ und 20°C.

1 Liter dieser Zellsuspension wird in die Druckfiltereinheit gemäß Zeichnung eingeführt und bei 2 bar Überdruck und 60°C einer Druckfiltration unterworfen.

Filteraufbau

| | |
|---|---|
| Filterstufe 2: | Polyurethanschaumstoff (Dichte: 60 kg/m$^3$, Porenweite: 0,55 mm, Schichthöhe: 50 mm) |
| Filterstufe 3: | Seidengaze (Porenweite: 0,006 mm, Schichthöhe: 0,5 mm) |
| Filterfläche: | 10,7 cm$^2$ |

Ergebnis:

Im wasserklaren Filtrat kann keine Zellmasse nachgewiesen werden, das Filtrat besitzt eine Viskosität bei 20°C und $D = 9s^{-1}$ von 129 mPas und einen Homoglucangehalt von 4,23 g/l, entsprechend einem Filtrationsverlust an Homoglucan von 0,48 Gew.%. Die Filtrationsrate beträgt 8972 l/m$^2$ h.

Beispiel 4

Zweistufige Druckfiltration zur Trennung von Glasmahlkugeln von Zellbruchstücken und viskosem Filtrat

60 g Zellfeuchtmasse des Pilzstammes Sclerotium glucanicum werden mit 50 ml Glasmahlkugeln (Durchmasser 0,25 mm) in 140 ml Wasser suspendiert und in einer Kugelmühle einem Zellaufschluß unterworfen. Die Glasmahlkugeln werden von der Zellaufschlußsuspension unter folgenden Bedingungen abgetrennt:

Filteraufbau

Filterstufe 2:
Polyurethanschaumstoff (Dichte: 60 kg/m$^3$, Porenweite: 0,55 mm, Schichthöhe: 55 mm)
Filterstufe 3:
Seidengaze (Porenweite: 0,05 mm, Schichthöhe: 0,5 mm)
Filterfläche:
10,7 cm$^2$
Filtrationsdruck:
1 bar Überdruck

Ergebnis:

Die Glasmahlkugeln werden quantitativ im Filtersystem zurückgehalten, während im Filrat zu über 96 Gew.% die Zelltrümmer vorliegen.

Beispiel 5

Zweistufige Druckfiltration zur Trennung einer wäßrigen Bakteriensuspension

2 Liter einer wäßrigen Bakteriensuspension von Streptomyces olivacens mit Zelltrockengewichtsanteil von 2,5 % wird durch Quervernetzung mit Glutardialdehyd flokkuliert mit einem mittleren Flokkendurchmasser von 0,2 bis 2,0 mm. Aus dieser Flockensuspension werden die Bakterienflocken quantitativ mit folgendem Filteraufbau abgetrennt:

Filteraufbau

Filterstufe 2:
Polyurethanschaumstoff (Dichte: 60 kg/m$^3$, Porenweite: 0,55 mm, Schichthöhe: 55 mm)
Filterstufe 3:
Seidengaze (Porenweite: 0,1 mm, Schichthöhe: 0,5 mm)
Filterfläche:
10,7 cm$^2$
Filtrationsdruck:
2 bar Überdruck

Ergebnis:

Im wasserklaren Filtrat kann keine Zellmasse nachgewiesen werden.

**Patentansprüche**

1. Verfahren zur Abtrennung fester Partikel verschiedener Größe aus viskosen Flüssigkeiten mit strukturviskosen Eigenschaften mittels Druckfiltration in mehreren Filterstufen verschiedener Schichthöhen, dadurch gekennzeichnet, daß die inhomogene oder homogenisierte Suspension, ohne oder nach nur geringer Verdünnung, der Druckfiltration unterworfen wird und dadurch die festen Partikel verschiedener Größen auf Filterschichten abgetrennt werden, wobei in der ersten Filterstufe mit einer Filterschicht aus einem dreidimensionalen Netzwerk aus Metallgewebe oder aus Kunststoffgeflecht oder aus Cellulosemasse der mittleren Porenweite von 1 bis 3 mm und in der 2. Filterstufe mit offenporigen Polyurethanschaumstoff einer Porenweite von 0,2 bis 0,8 mm und in der dritten Fillterstufe mit Seidengaze einer Maschenweite von 0,006 bis 0,2 mm getrennt wird.

2. Verfahren nach Anspruch 1, dadurch gekennzeichnet, daß die Abtrennung der festen Partikel, wie Mycelbruchstücke, aus der viskosen Flüssigkeit ohne die 1. Filterstufe nur in der 2. Filterstufe mit Polyurethanschaumstoff und in der 3. Filterstufe mit Seidengaze durchgeführt wird.

3. Verfahren nach den Ansprüchen 1 bis 2, dadurch gekennzeichnet, daß in einer Vorstufe mittels Mahlkörpern in einem Mahlsystem, beispielsweise in einer Kugelmühle, eine homogene Suspension der Partikel in der viskosen Flüssigkeit hergestellt, und danach die Mahlkörper, wie Glaskugeln mit einem Durchmesser von 0,25 mm, ohne die 1. Filterstufe in der 2. Filterstufe mit Polyurethanschaumstoff und danach in der 3. Filterstufe mit Seidengaze von der homogenen Phase, insbesondere einer Zellaufschlußsuspension, abgetrennt und gegebenenfalls in einer weiteren Filterstufe 3a mit einer Seidengaze kleinerer Porenweite die homogene Phase in feste Partikel und klare viskose Flüssigkeit getrennt wird.

4. Verfahren nach den Ansprüchen 1 bis 3, dadurch gekennzeichnet, daß in einer Vorstufe eine wäßrige Bakteriensuspension durch Quervernetzung, insbesondere mit Glutardialdehyd, auf einen mittleren Flockendurchmesser von 0,2 mm bis 2 mm flokkuliert, und danach die Bakterienflocken von der Flockensuspension ohne die 1. Filterstufe in der 2. Filterstufe mit Polyurethanschaumstoff, vorzugsweise mit einer Porenweite von 0,55 mm, und in der 3. Filterstufe mit Seidengaze, vorzugsweise mit einer Porenweite von 0,1 mm, abgetrennt werden.

5. Verfahren nach den Ansprüchen 1 bis 4, dadurch gekennzeichnet, daß der Überdruck 1 bis 10, insbesondere 1 bis 6 bar beträgt.

6. Verfahren nach den Ansprüchen 1 bis 5, dadurch gekennzeichnet, daß die Abtrennung der festen Partikel von der viskosen Flüssigkeit bei einer Temperatur der Suspension zwischen 20 °C bis 60 °C erfolgt.

7. Verfahren nach den Ansprüchen 1 bis 6, dadurch gekennzeichnet, daß die Abtrennung der festen Partikel aus einer mechanisch homogenisierten Zellsuspension einer Zellgrößenverteilung von 0,002 bis 0,005 mm mittleren Durchmessers erfolgt.

8. Verfahren nach den Ansprüchen 1 bis 7, dadurch gekennzeichnet, daß die Abtrennung der festen Partikel aus einer inhomogenen Zellsuspension mit Hyphenbruchstücken zwischen 0,005 mm bis 1 cm eines Mycelgehalts bis 10 gTG/l und/oder hoher Polymerkonzentration bis 12 gTG/l erfolgt, und gegebenenfalls die Filtrierbarkeit durch einen geringen Zusatz wäßriger Lösung herbeigeführt wird.

9. Verfahren nach den Ansprüchen 1 bis 8, dadurch gekennzeichnet, daß die Abtrennung der festen Partikel aus einer Zellsuspension der Viskosität bis 1200 mPas bei einer Scherrate von etwa D = 9 s$^{-1}$ bei 20 °C erfolgt.

10. Verfahren nach den Ansprüchen 1 bis 9, dadurch gekennzeichnet, daß die Schichthöhen in den Filterstufen folgende sind:

Stufe 1: 10 bis 100, insbesondere 40 bis 60 mm,
Stufe 2: 10 bis 100, insbesondere 40 bis 60 mm.
Stufe 3: 0,5 mm.

11. Verfahren nach Anspruch 10, dadurch gekennzeichnet, daß bei Filtration mit Filterstufe 1 in der Filterstufe 2 mit verminderter Schichthöhe filtriert wird.

12. Verfahren nach den Ansprüchen 1 bis 11, dadurch gekennzeichnet, daß in Filterstufe 2 Polyurethanschaumstoff der Dichte von 60 bis 80 kg/m$^3$ verwendet wird.

13. Verfahren nach den Ansprüchen 1 bis 12, dadurch gekennzeichnet, daß die Porenweite des Polyurethanschaumstoffs durch Kompressionsdruck variabel verkleinert wird.

14. Verfahren nach den Ansprüchen 1 bis 13, dadurch gekennzeichnet, daß die Filtration mit solchen Porenweiten durchgeführt wird, daß die Filtrationsrate 5.000 bis 10.000 l/m$^2$ h beträgt.

15. Verfahren nach den Ansprüchen 1 bis 14, dadurch gekennzeichnet, daß viskose Suspensionen aus Pilzstämmen, vorzugsweise aus der Züchtung von Sclerotium rolfsii, ATCC 15 205, oder aus Sclerotium Glucanicum, CBS 52 071, filtriert werden.

16. Anwendung des Verfahrens nach den Ansprüchen 1 bis 15 auf viskose Suspensionen aus Celluloid, Celluloseether, Cellulosechemiefasern, Fetten, Ölen, Gelatine, natürlichen und synthetischen Harzen, Klebstoffen, Dichtungsmassen, Pektinen, Stärke, Schmierstoffe und auf Polysaccharide aus Agar, Alginat, Corregeenan, Dextran.

17. Verfahren nach den Ansprüchen 1 bis 16, dadurch gekennzeichnet, daß zur Vermeidung oder Verringerung von Filtrationsverlusten eine oder mehrere Naßdecken aus wäßriger Lösung auf die Filtermassen der Filterstufen aufgegeben, und eine verdünnte Lösung des Filtrates, wie aus Homoglucan, gewonnen, und diese für die geringe Verdünnung der Suspension, oder zur Züchtung der Mikroorganismen, wie vorzugsweise Pilzkulturen, eingesetzt wird.

**Claims**

1. A process for separating solid particles of various sizes from viscous liquids having non-Newtonian properties by pressure filtration in a plurality of filter stages of different depths, which comprises subjecting the inhomogeneous or homogenized suspension, without or after only a little dilution, to a pressure filtration and thereby separating off the solid particles of various sizes on filter layers, the first filter stage utilizing a filter layer comprising a three-dimensional network, as of a metal wire or synthetic cloth or a cellulose material, from 1 to 3 mm in average pore size, and filter stage 2 utilizing an open-pored polyurethane foam from 0.2 to 0.8 mm in pore size, and the third filter stage utilizing silk gauze from 0.006 to 0.2 mm in mesh size.

2. A process as claimed in claim 1, wherein separation of the solid particles, such as mycelium fragments, from the viscous liquid is carried out without filter stage 1 only in filter stage 2, the polyurethane foam, and in filter stage 3, the silk gauze.

3. A process as claimed in claim 1 or 2, wherein a homogeneous suspension of particles in the viscous liquid is prepared in a preliminary stage by means of milling media in a milling system, for example a ball mill, and thereafter the milling media, such as glass balls 0.25 mm in diameter, are separated without filter stage 1 in filter stage 2, the polyurethane foam, and thereafter in filter stage 3, the silk gauze, from the homogeneous phase, in particular a suspension of disrupted cells, and optionally in a further filter stage 3a the homogeneous phase is separated into solid particles and clear viscous liquid using a silk gauze of smaller mesh size.

4. A process as claimed in any of claims 1 to 3, wherein in a preliminary stage an aqueous bacterial suspension is flocculated by crosslinking, in particular with glutardialdehyde, to an average floc diameter of from 0.2 mm to 2 mm and thereafter the bacterial flocs are separated from the flocculated suspension without filter stage 1 in filter stage 2, the polyurethane foam, preferably of a pore size of 0.55 mm, and in filter stage 3, the silk gauze, preferably of a pore size of 0.1 mm.

5. A process as claimed in any of claims 1 to 4, wherein the positive pressure is from 1 to 10, in particular from 1 to 6, bar.

6. A process as claimed in any of claims 1 to 5, wherein the solid particles are separated from the viscous liquid at a temperature of the suspension of from 20°C to 60°C.

7. A process as claimed in any of claims 1 to 6, wherein the solid particles are separated from a mechanically homogenized cell suspension having a cell size distribution from 0.002 to 0.005 mm average diameter.

8. A process as claimed in any of claims 1 to 7, wherein the solid particles are separated from an inhomogeneous cell suspension containing hyphae fragments of from 0.005 mm to 1 cm and having a mycelium content of up to 10 g of dry matter/1 or high polymer concentration up to 12 g of dry matter/1, and if necessary filterability is brought about by adding a small amount of aqueous solution.

9. A process as claimed in any of claims 1 to 8, wherein the solid particles are separated from a cell suspension having a viscosity of up to 1200 mPas at a shear rate of about D = 9 s$^{-1}$ at 20°C.

10. A process as claimed in any of claims 1 to 9, wherein the filter stage depths are as follows:
    Stage 1:  from 10 to 100, in particular from 40 to 60, mm
    Stage 2:  from 10 to 100, in particular from 40 to 60, mm
    Stage 3:  0.5 mm.

11. A process as claimed in claim 10, wherein, if filtration is carried out with filter stage 1, the depth of filter stage 2 is reduced.

12. A process as claimed in any of claims 1 to 11, wherein filter stage 2 comprises a polyurethane foam from 60 to 80 kg/m$^3$ in density.

**13.** A process as claimed in any of claims 1 to 12, wherein the pore size of the polyurethane foam is variably reduced in size by compression.

**14.** A process as claimed in any of claims 1 to 13, wherein the filtration is carried out with such pore sizes that the filtration rate is from 5,000 to 10,000 $1/m^2$ h.

**15.** A process as claimed in any of claims 1 to 14, wherein viscous suspensions of fungus strains, preferably from culturing Sclerotium rolfsii, ATCC 15 205, or Sclerotium glucanicum CBS 52 071, are filtered.

**16.** Use of the process as claimed in any of claims 1 to 15 on viscous suspensions of celluloid, cellulose ethers, cellulosic fibers, fats, oils, gelatin, natural and synthetic resins, adhesives, sealants, pectins, starch, lubricants and on polysaccharides from agar, alginate, carregeen or dextran.

**17.** A process as claimed in any of claims 1 to 16, wherein, to avoid or reduce filtration losses, one or more wet blankets of aqueous solution are applied to the filter materials of the filter stages, and a dilute solution of the filtrate, as of homoglucan, is isolated and used for slightly diluting the suspension or for culturing microorganisms, such as, preferably, fungal cultures.

**Revendications**

**1.** Procédé de séparation de particules solides de différentes tailles de liquides visqueux à comportement non newtonien par filtration sous pression dans plusieurs étages de hauteurs de couche différentes, caractérisé par le fait que la suspension non homogène ou homogénéisée est soumise à la filtration sous pression sans dilution ou après une faible dilution et les particules solides de différentes tailles sont ainsi séparées sur des couches filtrantes, la séparation étant faite, dans le premier étage, avec une couche filtrante constituée d'un réseau tridimensionnel en tissu métallique ou en treillis de matière plastique ou en matière cellulosique ayant une taille moyenne de pores de 1 à 3 mm, dans le deuxième étage, avec du polyuréthanne alvéolaire à alvéoles ouverts ayant une taille de pores de 0,2 à 0,8 mm et, dans le troisième étage, avec de la gaze de soie ayant une ouverture de maille de 0,006 à 0,2 mm.

**2.** Procédé selon la revendication 1, caractérisé par le fait que la séparation des particules solides, telles que fragments de mycélium, du liquide visqueux est faite, sans le premier étage, seulement dans le deuxième étage avec du polyuréthanne alvéolaire et, dans le troisième étage, avec de la gaze de soie.

**3.** Procédé selon les revendications 1 et 2, caractérisé par le fait qu'une suspension homogène des particules dans le liquide visqueux est produite dans une étape préliminaire au moyen de corps broyeurs dans un système de broyage, par exemple dans un broyeur à billes et, ensuite, les corps broyeurs, tels que billes de verre de 0,25 mm de diamètre, sont séparés de la phase homogène, en particulier d'une suspension de décomposition cellulaire, sans le premier étage de filtration, dans le deuxième étage de filtration avec du polyuréthanne alvéolaire et ensuite dans le troisième étage de filtration avec de la gaze de soie, et éventuellement, la phase homogène est divisée en particules solides et liquide visqueux clair dans un étage supplémentaire de filtration 3a avec une gaze de soie ayant une plus petite taille de pores.

**4.** Procédé selon les revendications 1 à 3, caractérisé par le fait que, dans une étape préliminaire, une suspension aqueuse de bactéries est soumise à floculation à un diamètre moyen des flocons de 0,2 à 2 mm par réticulation transversale, en particulier au glutardialdéhyde et, ensuite, les flocons de bactéries sont séparés de la suspension de flocons, sans le premier étage de filtration, dans le deuxième étage de filtration avec du polyuréthanne alvéolaire ayant de préférence une taille de pores de 0,55 mm et, dans le troisième étage de filtration, avec de la gaze de soie ayant de préférence une taille de pores de 0,1 mm.

**5.** Procédé selon les revendications 1 à 4, caractérisé par le fait que la surpression est de 1 à 10 bar, en particulier de 1 à 6 bar.

**6.** Procédé selon les revendications 1 à 5, caractérisé par le fait que la séparation des particules solides du liquide visqueux est faite à une température de la suspension comprise entre 20 degrés C et 60 degrés C.

**7.** Procédé selon les revendications 1 à 6, caractérisé par le fait qu'on sépare les particules solides d'une suspension cellulaire homogénéisée mécaniquement ayant une distribution de tailles de cellules de 0,002 à 0,005 mm de diamètre moyen.

**8.** Procédé selon les revendications 1 à 7, caractérisé par le fait qu'on sépare les particules solides d'une suspension cellulaire non homogène à fragments d'hyphes entre 0,005 mm et 1 cm, ayant une teneur en mycélium allant jusqu'à 10 g (à sec)/1 et/ou une haute concentration en polymère allant jusqu'à 12 g (à sec)/1, et éventuellement produit la filtrabilité par une faible addition de solution aqueuse.

**9.** Procédé selon les revendications 1 à 8, caractérisé par le fait qu'on sépare les particules solides d'une suspension cellulaire ayant une viscosité allant jusqu'à 1200 mPa.s.à 20 degrés C, avec un gradient de vitesse de cisaillement d'environ 9 s$^{-1}$.

**10.** Procédé selon les revendications 1 à 9, caractérisé par le fait que les hauteurs de couches dans les étages de filtration sont les suivantes :

Etage 1 : 10 à 100 mm, en particulier 40 à 60 mm
Etage 2 : 10 à 100 mm, en particulier 40 à 60 mm
Etage 3 : 0,5 mm

**11.** Procédé selon la revendication 10, caractérisé par le fait qu'un cas de filtration avec l'étage 1, on filtre dans l'étage 2 avec une hauteur de couche réduite.

**12.** Procédé selon les revendications 1 à 11, caractérisé par le fait que dans l'étage de filtration 2 est utilisé du polyuréthanne alvéolaire ayant une masse volumique de 60 à 80 kg/m$^3$.

**13.** Procédé selon les revendications 1 à 12, caractérisé par le fait qu'on réduit de manière variable la taille des pores du polyuréthanne alvéolaire en comprimant celui-ci.

**14.** Procédé selon les revendications 1 à 13, caractérisé par le fait que la filtration est faite avec des tailles de pores telles que le débit de filtration soit de 5000 à 10 000 1/m$^2$.h.

**15.** Procédé selon les revendications 1 à 14, caractérisé par le fait qu'on filtre des suspensions visqueuses de souches de champignons, de préférence provenant de la culture de Sclerotium rolfsii, ATCC 15 205, ou provenant de Sclerotium Glucanicum, CBS 52 071.

**16.** Application du procédé selon les revendications 1 à 15 à des suspensions visqueuses de celluloïd, d'éther cellulosique, de fibres cellulosiques synthétiques, de graisses, d'huiles, de gélatine, de résines naturelles ou synthétiques, d'adhésifs, de matière d'étanchéité, de pectines, d'amidon, de lubrifiants et à des polysaccharides d'agar-agar, d'alginate, de corregeenane, de dextrane.

**17.** Procédé selon les revendications 1 à 16, caractérisé par le fait que pour éviter ou réduire les pertes par filtration, on applique sur les matières filtrantes des étages de filtration une ou plusieurs couvertures humides de solution aqueuse, et produit une solution diluée du filtrat, par exemple d'homoglycanne, et utilise celle-ci pour la faible dilution de la suspension ou pour la culture des micro-organismes, de préférence des cultures de champignons.